# EUROPÄISCHE PATENTSCHRIFT

(11) **EP 2 296 726 B1**
(45) Veröffentlichungstag und Bekanntmachung des Hinweises auf die Patenterteilung: **27.01.2016**
(21) Anmeldenummer: 09772145.0
(22) Anmeldetag: 30.06.2009
(51) Int. Cl.: A61M 1/28

(54) **VORRICHTUNG ZUR PERITONEALDIALYSE**
DEVICE FOR USE WITH PERITONEAL DIALYSIS
APPAREIL POUR LA DIALYSE PERITONEALE

(30) Priorität: 04.07.2008 DE 102008031660
(43) Veröffentlichungstag der Anmeldung: 23.03.2011
(73) Patentinhaber: Fresenius Medical Care Deutschland GmbH, 61352 Bad Homburg (DE)
(72) Erfinder: HEDMANN, Frank, 97332 Volkach (DE); KLATTE, Stephan, 31582 Nienburg (Weser) (DE)
(74) Vertreter: Laufhütte, Dieter
(86) Internationale Anmeldenummer: PCT/EP2009/004720
(87) Internationale Veröffentlichungsnummer: WO 2010/000445

(56) Entgegenhaltungen:
- WO-A1-99/06082
- WO-A2-2009/091706
- US-A- 3 809 243
- US-A1- 2004 019 313
- US-A1- 2008 045 884

## Beschreibung

Die Erfindung betrifft eine Vorrichtung zur Peritonealdialyse nach dem Oberbegriff des Anspruchs 1.

Bei der Peritonealdialyse (PD) wird eine Dialyselösung (Dialysat) in die Bauchhöhle eines Patienten eingefüllt. Nach einer gewissen Verweilzeit, während der ein Stoffaustausch über das Peritoneum des Patienten zwischen Dialysat und Blut stattfindet, wird das nun "verbrauchte" Dialysat gegen frisches Dialysat ausgetauscht. Hierzu wird das verbrauchte Dialysat üblicherweise in einen Beutel abgelassen. Das Dialysat verändert während der Verweilzeit in der Bauchhöhle seine Farbe. Neben einer Verfärbung des Dialysats ist häufig auch eine Trübung zu beobachten. Im Normalfall sollte das Dialysat den Farbton von Apfelsaft haben. Die entsprechend auftretende Verfärbung bzw. Trübung wird beim verbrauchten Dialysat durch den Arzt, den Dialyseverantwortlichen oder den Patienten selbst nach dem Auslaufen einer Sichtkontrolle unterzogen. Anhand der Trübung erfolgt eine Beurteilung, ob das verbrauchte Dialysat eine übliche, normale Verfärbung aufweist. Wird nun die Verfärbung als nicht normal eingestuft, werden üblicherweise weitere Untersuchungen zur Ursachenforschung initiiert. Verfärbungen, die nicht in dem üblich erwarteten Normbereich liegen, können auf unterschiedliche krankhafte Ursachen hinweisen, wie beispielsweise eine vermehrte Eiweißausscheidung, eine Blutausscheidung oder sie könnte beispielsweise auf eine angehende Peritonitis (Bauchfellentzündung) hinweisen. In jedem Fall ist es bei nicht üblichen Verfärbungen angeraten, einen Arzt zu konsultieren, um gegebenenfalls weitergehende Untersuchungen zu veranlassen.

Problematisch bei der Sichtkontrolle ist die subjektive Wahrnehmung des Beobachters, insbesondere des nicht geübten Patienten. Hier kann aufgrund subjektiver und sich ändernder Beurteilungsmaßstäbe nicht immer mit gleichbleibender Sicherheit erkannt werden, ob es sich um eine vergleichsweise eher normale oder schon anormale Verfärbung des Dialysats im Auslauf aus der Bauchhöhle handelt.

Aus der WO 99/06082 A1 ist bereits ein Verfahren zur Durchführung einer Peritonealdialysebehandlung sowie eine zugehörige Peritonealdialysebehandlungsmaschine bekannt. Dabei wird das gebrauchte Dialysat mittels eines Turbidimeters überprüft, um beispielsweise eine beginnende Peritonitis erkennen zu können.

Aus der US 2004/0019313 A1 ist ebenfalls eine Behandlungsmaschine zur Durchführung einer Peritonealdialysebehandlung bekannt. Mittels Sensoren kann die Trübung des Dialysats bestimmt werden. Ferner weist die Behandlungsmaschine zur Durchführung der Peritonealdialysebehandlung einen Touchscreen auf.

Aus der US 2008/0045884 A1 ist ebenfalls eine Behandlungsmaschine zur Durchführung einer Peritonealdialysebehandlung bekannt, bei der mittels Trübungsmessung eine Peritonitisfrüherkennung möglich ist.

Die US 3,809,243 offenbart ein Trübungsmessgerät für Dialysemaschinen.

Die Aufgabe der Erfindung ist es nun, eine gattungsgemäße Vorrichtung zur Peritonealdialyse derart weiterzubilden, dass die Objektivität bei der Beurteilung der Verfärbung bzw. Trübung des Dialysats verbessert wird und eine maschinelle Unterstützung in der Auswertung der Trübung an die Hand gegeben wird.

Diese Aufgabe wird erfindungsgemäß durch eine Vorrichtung mit den Merkmalen des Anspruchs 1 gelöst. Demnach wird eine Vorrichtung zur Peritonealdialyse mit einer Einrichtung zur regelmäßigen Abgabe und Wiederaufnahme von Dialysat (Cycler) bereitgestellt, die eine Leitung mit einem Katheter umfaßt. Diese Einrichtung weist nun erfindungsgemäß ein Mittel auf, über welches die Farbe bzw. Trübung des Dialysats bestimmbar ist. Aufgrund dieser erfindungsgemäßen Lösung kann der Bediener, das heißt der Arzt, der Dialyseverantwortliche oder sogar der Patient selbst die Trübung des zu beurteilenden aktuellen Dialysats mit objektiven Mitteln bestimmen bzw. bestimmen lassen.

Das Mittel zur Farb- und Trübungsbestimmung weist erfindungsgemäß ein Display, vorzugsweise in Form eines Touchscreens, auf. Auf dem Display ist erfindungsgemäß die Farbe bzw. Trübung in einem eigens dafür vorgesehenen Fenster darstellbar und auswählbar.

Bevorzugte Ausgestaltungen der Erfindung ergeben sich aus den sich an den Hauptanspruch anschließenden Unteransprüchen.

Demnach können die über das Mittel erhaltenen Farb- bzw. Trübungswerte des Dialysats abspeicherbar sein. Hierzu können die Farb- und Trübungswerte in einem in der Einrichtung vorgesehenen Speicher ablegbar sein.

Gemäß einer anderen vorteilhaften Ausgestaltung können die Farb- und Trübungswerte zusammen mit alphanumerischen Daten, beispielsweise den Patientendaten, auf einem herausnehmbaren Speichermedium, beispielsweise einem Speicherchip auf der Patientenkarte, ablegbar sein.

Dabei kann auf dem Display ein Farbspektrum darstellbar sein, aus dem die zutreffende Farbe bzw. Trübung durch den Bediener auswählbar ist. Um einen unmittelbaren Vergleich zwischen dem Dialysat und der auf dem Display angegebenen Farbskala zu ermöglichen, ist es besonders vorteilhaft, wenn im Bereich des Displays ein Beleuchtungsfeld vorgesehen ist, über das eine Drainageleitung bzw. Küvette, die jeweils mit Dialysat gefüllt sind, beleuchtbar sind. Anhand des sich ergebenden Farb- bzw. Trübungswerts in dem Dialysat kann der Bediener innerhalb der sich ergebenden Farb- bzw. Trübungswelrtskala auf dem Display den zutreffenden Wert auswählen und eingeben.

Die Vorrichtung kann vorzugsweise im Bereich des Displays ein Alarmfeld aufweisen, das bei einer vorbestimmten Abweichung des bestimmten Farb- bzw. Trübungswertes von einem vorgegebenen Farb- bzw. Trübungswert einen Alarm ausgibt. Dieser Alarm kann alleine als optischer Alarm auf dem Display ausgegeben werden. Alternativ ist es aber auch denkbar, dass hier eine zusätzliche akustische Alarmglocke oder ein sonstiges Alarmsignal ertönt. Gegebenenfalls kann auch ein akustisches Alarmsignal alleine ohne Anzeigen auf dem Display ausgegeben werden. Die Ausgabe des Alarms verlangt, dass im vorliegenden Fall ein Arzt konsultiert werden sollte, durch den dann weitere Untersuchungen erfolgen können. Schließlich können nach einer bevorzugten Ausgestaltung auf dem Display die Farb- bzw. Trübungswerte vergangener Messungen im Vergleich zueinander anzeigbar sein. Hier kann der Arzt beispielsweise aus der Trübungshistorie und den hinterlegten Trübungswerten bzw. Farbwerten bereits ein Anhaltspunkt auf die vorliegenden Komplikationen erhalten und so eine gezielte Untersuchung in die Wege leiten.

Die Erfindung bezieht sich auch auf ein entsprechendes Verfahren, wie es in den Ansprüchen beansprucht wurde.

Weitere Einzelheiten und Vorteile der Erfindung werden anhand eines in der Zeichnung dargestellten Ausführungsbeispieles näher erläutert. Es zeigen:

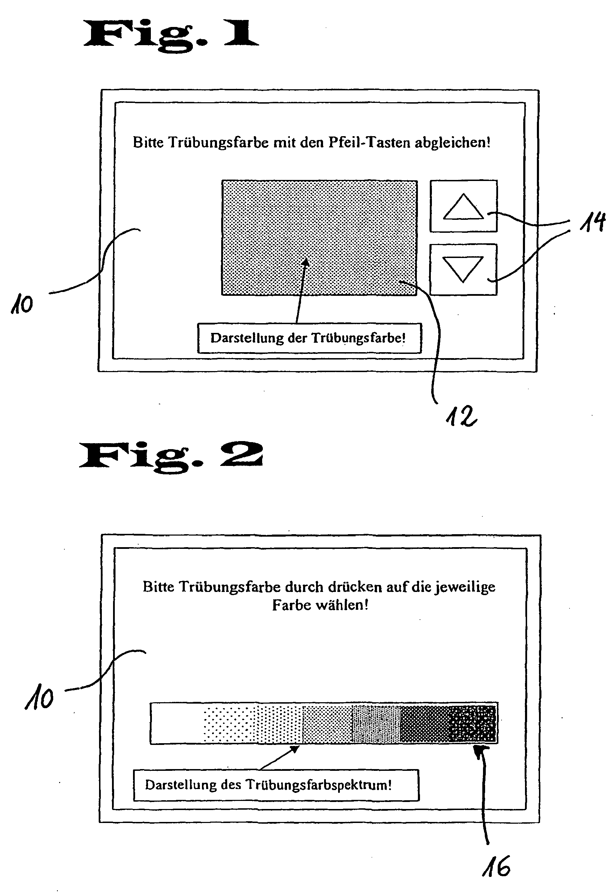

- Figuren 1 - 5:: unterschiedliche Ausgestaltungen eines Displays einer erfindungsgemäßen Vorrichtung.

Die Figur 1 zeigt ein Display 10, das an einer Einrichtung zur regelmäßigen Abgabe und Wiederaufnahme von Dialysat innerhalb einer Vorrichtung zur Peritonealdialyse vorgesehen kann. Anhand dieses Displays kann die Farbe des abdrainierten Dialysats nach einer Peritonealdialysebehandlung bestimmt werden. Hierzu ist auf dem Display 10, welches als Touchscreen ausgebildet ist, ein Fenster 12 vorgesehen, in welchem eine typische Farbe bzw. ein typischer Trübungsgrad dargestellt ist, der der Farbe eines "verbrauchten" Dialysats entsprechen kann. Über die mittels Berührung auf dem Touchscreen aktivierbaren Pfeiltasten 14 kann der Bediener, das heißt der Arzt, der Dialyseverantwortliche oder der Patient selbst den Farbwert aus einer entsprechenden Skala, die in einem dem Display 10 zugeordneten Speicher abgelegt ist, verändern, um so denjenigen Wert auszuwählen, der der aktuellen Farbe bzw. dem aktuellen Trübungsgrad des Dialysats entspricht. Bei der Feststellung einer Übereinstimmung zwischen der im Fenster 12 dargestellten Farbe bzw. dem dargestellten Trübungsgrad und dem Dialysat kann durch entsprechendes Berühren des Fensters 12 der dort gerade angezeigte Wert gespeichert werden. Die hier anhand eines Touchscreens erläuterte Bedienbarkeit kann selbstverständlich auch bei vorsehen eines einfachen Displays 10 ohne Touchscreenbedienung durch andere Eingabemöglichkeiten wie eine übliche Computertastatur oder eine Computermaus erfolgen.

In der Figur 2 ist eine alternative Ausführungsvariante gezeigt, bei der das Display 10 in seinem Ausgabefenster 16 nicht nur einen bestimmten Farb- bzw. Trübungswert angibt, sondern ein gesamtes Trübungsfarbspektrum. Bei Ausführung des Displays als Touchscreen kann durch entsprechendes Berühren des Spektrums in dem Farb- bzw. Trübungsbereich, der demjenigen des Dialysats entspricht, der Wert ausgewählt und gespeichert werden.

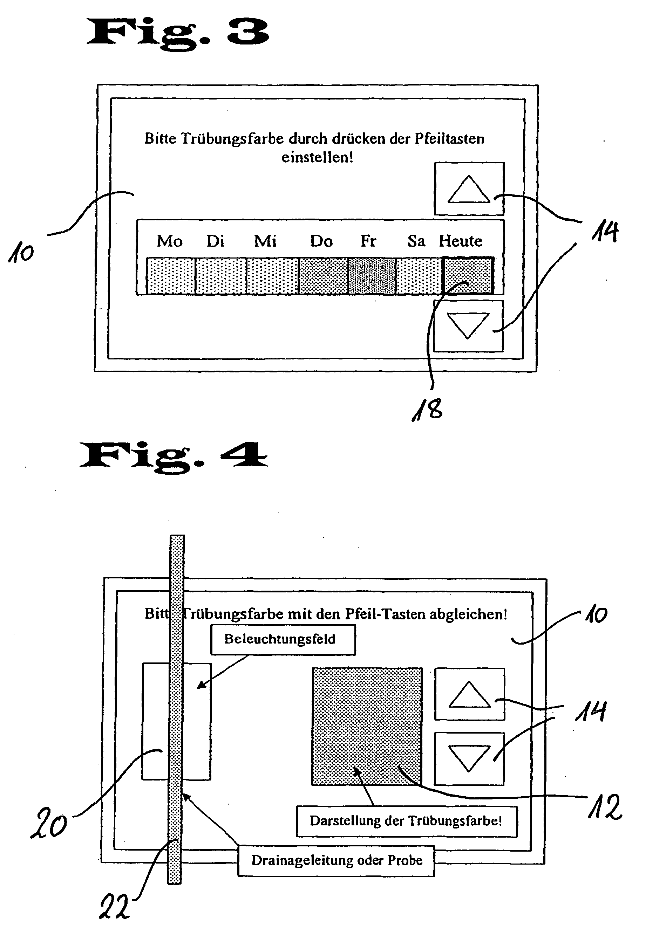

Eine weitere alternative Ausgestaltung eines Displays, wie es in Figur 2 dargestellt ist, das jedoch eine etwas unterschiedliche Bedienmimik aufweist, ist in Figur 3 dargestellt. Hier wird ähnlich der Ausführungsvariante gemäß Figur 1 in einem Fenster 18 über Pfeiltasten 14, die im Touchscreen vorgesehen sind, der aktuell für die Dialysebehandlung festgestellte Wert eingegeben. Aufgrund eines internen Speichers wird dann dieser Wert abgespeichert und am Folgetag unter dem entsprechenden Wochentag im Fenster 16 angegeben, so dass sich hier im dargestellten Ausführungsbeispiel eine Wochenhistorie ergibt. Die für den behandelnden Arzt Aufschluß über eventuelle Veränderungen des Dialysats gibt und damit Rückschlüsse auf möglicherweise vorhandene krankhafte Veränderungen zuläßt.

In Figur 4 ist ein Display 10 dargestellt, welches im rechten Teil im wesentlichen der Ausführungsform gemäß Figur 1 entspricht und die entsprechende Funktionalität aufweist. Um das Dialysat unmittelbar vergleichen zu können, ist im Display aber noch ein zusätzliches Beleuchtungsfeld 20 vorgesehen, vor dem im hier dargestellten Ausführungsbeispiel eine durchsichtige Drainageleitung 22 angeordnet ist, die aufgrund ihrer Durchsichtigkeit eine Durchleuchtung ermöglicht. Somit kann das durch diese Drainageleitung geführte Dialysat vom Bediener in seiner Verfärbung unmittelbar mit der Darstellung der im Fenster 12 ausgewählten Farbe bzw. mit dem dort dargestellten Trübungsgrad erfolgen. Hierdurch wird die Feststellung des aktuellen Farbwertes bzw. Trübungswertes des Dialysats weitgehend objektiviert.

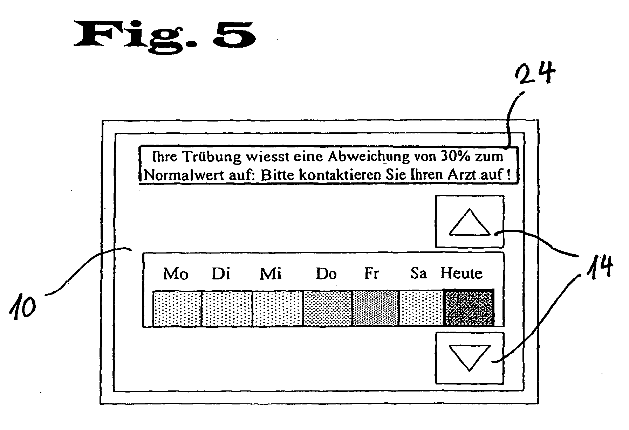

In der Darstellung gemäß Figur 5 ist eine Ausführungsvariante eines Displays 10 gezeigt, die weitgehend derjenigen gemäß Figur 3 entspricht. Hier ist allerdings noch ein zusätzliches Alarmanzeigefeld 24 integriert, in welchem ein alphanumerisches Alarmsignal ausgegeben werden kann, wenn der aktuell festgestellte Farb- bzw. Trübungswert des Dialysats über ein vorbestimmtes Maß von dem vorgegebenen Trübungswert abweicht. Diese optische alphanumerische Alarmanzeige kann wahlweise auch akustisch unterstützt werden.

## Patentansprüche

1. Vorrichtung zur Peritonealdialyse mit einer Einrichtung zur regelmäßigen Abgabe und Wiederaufnahme von Dialysat, wobei die Einrichtung ein Mittel aufweist, über welches die Farbe bzw. Trübung des Dialysats bestimmbar ist, und wobei das Mittel zur Farb- und Trübungsmessung ein Display, vorzugsweise in Form eines Touchscreens, aufweist,
**dadurch gekennzeichnet,**
**dass** auf dem Display die Farbe bzw. Trübung in einem eigens dafür vorgesehenen Fenster darstellbar und auswählbar ist.

2. Vorrichtung nach Anspruch 1, **dadurch gekennzeichnet, dass** die über das Mittel erhaltenen Farb- bzw. Trübungswerte des Dialysats abspeicherbar sind.

3. Vorrichtung nach Anspruch 2, **dadurch gekennzeichnet, dass** die Farb- und Trübungswerte in einem in der Einrichtung vorgesehen Speicher ablegbar sind.

4. Vorrichtung nach Anspruch 2, **dadurch gekennzeichnet, dass** die Farb- und Trübungswerte zusammen mit alphanumerischen Daten, beispielsweise den Patientendaten, auf einem herausnehmbaren Speichermedium, beispielsweise einem Speicherchip, ablegbar ist.

5. Vorrichtung nach einem der Ansprüche 1 bis 4, **dadurch gekennzeichnet, dass** auf dem Display ein Farbspektrum darstellbar ist, aus dem die zutreffende Farbe bzw. Trübung auswählbar ist.

6. Vorrichtung nach einem der vorangehenden Ansprüche, **dadurch gekennzeichnet, dass** vorzugsweise im Bereich des Displays ein Beleuchtungsfeld vorgesehen ist, über das eine Drainageleitung bzw. Küvette, die jeweils mit Dialysat gefüllt sind, beleuchtbar sind.

7. Vorrichtung nach einem der vorangehenden Ansprüche, **dadurch gekennzeichnet, dass** vorzugsweise auf dem Display ein Alarmfeld vorgesehen ist, das bei einer vorbestimmten Abweichung des bestimmten Farb- bzw. Trübungswertes einen Alarm ausgibt.

8. Vorrichtung nach einem der vorangehenden Ansprüche, **dadurch gekennzeichnet, dass** auf dem Display die Farb- bzw. Trübungswerte vergangener Messungen im Vergleich zueinander anzeigbar sind.

9. Verfahren zur Bestimmung der Farbe bzw. der Trübung eines Dialysats, wobei die Farb- und Trübungswerte vorzugsweise zusammen mit alphanumerischen Werten, beispielsweise den Patientendaten, auf einem Speicher oder einem herausnehmbaren Speichermedium gespeichert werden, **dadurch gekennzeichnet, dass** auf einem Display die Farbe bzw. Trübung in einem eigens dafür vorgesehenen Fenster darstellbar und auswählbar ist.

10. Verfahren nach Anspruch 9, **dadurch gekennzeichnet, dass** auf einem Display ein Farbspektrum dargestellt wird und dass das Dialysat durch eine durchsichtige Leitung geleitet wird oder in eine durchsichtige Küvette gefüllt wird, die vorzugsweise durch ein Beleuchtungsfeld des Displays durchleuchtet werden, um einen Vergleich und eine Auswahl des zutreffenden Farb- bzw. Trübungswert zu ermöglichen.

11. Verfahren nach Anspruch 9 oder 10, **dadurch gekennzeichnet, dass** bei Abweichung von einem Vergleichsfarbwert um mehr als einen vorbestimmten Wert ein Alarm ausgelöst wird.

12. Verfahren nach einem der Ansprüche 9 bis 11, **dadurch gekennzeichnet, dass** auf dem Display die Farb- und Trübungswerte der vergangenen Messungen angezeigt werden.

## Claims

1. A device for peritoneal dialysis with a means for the regular discharge and reuptake of dialysate, wherein the device includes a means via which the color and turbidity of the dialysate can be determined, and wherein the means for color and turbidity measurement includes a display, preferably in the form of a touch screen,
**characterized in**
**that** on the display the color and turbidity can be represented and selected in a window especially provided for this purpose.

2. The device according to claim 1, **characterized in that** the color and turbidity values of the dialysate obtained by the means can be stored.

3. The device according to claim 2, **characterized in that** the color and turbidity values can be stored in a memory provided in the device.

4. The device according to claim 2, **characterized in that** the color and turbidity values can be stored together with alphanumeric data, for example the patient data, on a removable storage medium, for example a memory chip.

5. The device according to any of claims 1 to 4, **characterized in that** a color spectrum can be represented on the display, from which the applicable color and turbidity can be selected.

6. The device according to any of the preceding claims, **characterized in that** preferably in the region of the display an illumination field is provided, via which a drainage line or cuvette, which each are filled with dialysate, can be illuminated.

7. The device according to any of the preceding claims, **characterized in that** on the display an alarm field preferably is provided, which issues an alarm in the case of a predetermined deviation of the color and turbidity value determined.

8. The device according to any of the preceding claims, **characterized in that** the color and turbidity values of past measurements can be indicated on the display in comparison to each other.

9. A method for determining the color and turbidity of a dialysate, wherein the color and turbidity values preferably are stored together with alphanumeric values, for example the patient data, on a memory or on a removable storage medium, **characterized in that** on a display the color and turbidity can be represented and selected in a window especially provided for this purpose.

10. The method according to claim 9, **characterized in that** a color spectrum is represented on a display and that the dialysate is passed through a transparent line or filled into a transparent cuvette, which preferably are transilluminated by an illumination field of the display, in order to provide for a comparison and a selection of the applicable color and turbidity value.

11. The method according to claim 9 or 10, **characterized in that** in the case of a deviation from a comparative color value by more than a predetermined value an alarm is triggered.

12. The method according to any of claims 9 to 11, **characterized in that** the color and turbidity values of the past measurements are indicated on the display.

## Revendications

1. Dispositif pour la dialyse péritonéale avec un moyen de distribution et de reprise régulières de dialysat, dans lequel le dispositif comporte un moyen par lequel la couleur, respectivement, la turbidité, du dialysat peuvent être déterminées, et dans lequel le moyen présente un écran pour mesurer la couleur et la turbidité, de préférence sous la forme d'un écran tactile,
**caractérisé en ce**
**que** sur l'écran, la couleur, respectivement, la turbidité, peuvent être représentées et sélectionnées dans une fenêtre particulièrement prévue à cet effet.

2. Dispositif selon la revendication 1, **caractérisé en ce que** les valeurs de la couleur, respectivement, de la turbidité, du dialysat peuvent être mémorisées par l'intermédiaire du moyen.

3. Dispositif selon la revendication 2, **caractérisé en ce que** les valeurs de la couleur et de la turbidité peuvent être mémorisées dans une mémoire prévue dans le dispositif.

4. Dispositif selon la revendication 2, **caractérisé en ce que** les valeurs de la couleur et de la turbidité peuvent être mémorisées conjointement avec des données alphanumériques, par exemple, les données du patient, sur un support de stockage amovible, par exemple, une puce mémoire.

5. Dispositif selon l'une des revendications 1 à 4, **caractérisé en ce qu'**un spectre de couleurs peut être présenté sur l'écran, à partir duquel la couleur, respectivement, la turbidité, applicables peuvent être sélectionnées.

6. Dispositif selon l'une des revendications précédentes, **caractérisé en ce que**, de préférence dans la zone de l'écran, un champ d'éclairement est prévu, par lequel un conduit de drainage, respectivement, une cuvette, qui sont chacun remplis de dialysat peuvent être éclairés.

7. Dispositif selon l'une des revendications précédentes, **caractérisé en ce qu'**une zone d'alarme est de préférence prévue sur l'écran qui émet une alarme pour une déviation prédéterminée des valeurs définies de la couleur, respectivement, de la turbidité.

8. Dispositif selon l'une des revendications précédentes, **caractérisé en ce que** les valeurs de la couleur, respectivement, de la turbidité, d'anciennes prises de mesures peuvent être montrées sur l'écran à titre de comparaison.

9. Procédé de détermination de la couleur, respectivement, de la turbidité, d'un dialysat, dans lequel les valeurs de la couleur, respectivement, de la turbidité, sont mémorisées de préférence conjointement avec des valeurs alphanumériques, par exemple, des données du patient, sur une mémoire ou un support de stockage amovible, **caractérisé en ce que** sur l'écran, la couleur, respectivement, la turbidité, peuvent être représentées et sélectionnées dans une fenêtre particulièrement prévue à cet effet.

10. Procédé selon la revendication 9, **caractérisé en ce qu'**un spectre de couleurs est représenté sur un écran et que le dialysat est mené à travers un conduit transparent ou est rempli dans une cuvette transparente, qui sont éclairés de préférence par un champ d'éclairement de l'écran, afin de permettre une comparaison et une sélection des valeurs applicables de couleur, respectivement, de turbidité.

11. Procédé selon les revendications 9 ou 10, **caractérisé en ce que**, pour une déviation d'une valeur de la couleur en comparaison supérieure à une valeur prédéterminée, une alarme est déclenchée.

12. Procédé selon l'une des revendications 9 à 11, **caractérisé en ce que** les valeurs de la couleur et de la turbidité des anciennes mesures sont indiquées sur l'écran.
